# EUROPEAN PATENT APPLICATION

(11) **EP 1 229 097 A1**
(43) Date of publication of application: **07.08.2002**
(21) Application number: 99956375.2
(22) Date of filing: 13.08.1999
(51) Int. Cl.: C09K 5/16, D04H 3/16, A61F 7/03

(54) **COMPOSITE EXOTHERMIC NON-WOVEN MATERIAL AND METHODS FOR THE PRODUCTION AND ACTIVATION THEREOF**

(71) Applicant: The Procter & Gamble Company, Cincinnati, Ohio 45202 (US)
(72) Inventor: DYATLOV, Valery Alexandrovich, Moscow, 119034 (RU); HARTMUT, Hoehne, D-65842 Schwalbach am Taunus (DE); VINOGRADOV, Valentin Antonovich, Moscow, 123007 (RU); KRENEV, Vladimir Alexandrovich, Moscow, 109377 (RU); DROBOT, Nataliya Fedorovna, Moscow, 125319 (RU); GAVRICHEV, Konstantin Sergeevich, Moscow, 117334 (RU); BABIEVSKAYA, Irina Zinovievna, Moscow, 117454 (RU); NOSKOVA, Olga Anatolievna, Moskovskaya obl., 144007 (RU); BAZHENOVA, Elena Vladimirovna, Moscow, 127238 (RU); VASILIEV, Viktor Sergeevich, Moscow, 12542 (RU); TSIPERMAN, Vladimir Leonidovich, Tver, 171000 (RU); IDIATULOV, Rafet Kutuzovich, Tver, 171000 (RU)
(74) Representative: Cabinet Hirsch
(86) International application number: RU9900295
(87) International publication number: WO0112749

(57) **Abstract**

The present invention relates to an exothermic nonwoven composite material on the base of a polymer fiber with a filler capable of releasing heat upon contact with air, with the polymer:filler (weight) ratio equal to 1:1 - 1:4, preferably 1:3 - 1:4. The filler may be a finely dispersed powdered metal iron having a particle size of not more than 20 microns or a powdered mixture of metal iron and activated carbon or the aforesaid mixture with the addition of vermiculite or the aforesaid mixture with the addition of water, for example, powdered metal iron - 0.717 g, activated carbon - 0.151 g, vermiculite - 0.026 g, water - 0.106 g. The polymer fiber is, for example, a copolymer of acrylonitrile and methyl acrylate, taken in the ratio, % by weight, of 95:5, respectively. A method is also proposed for preparing an exothermic nonwoven composite material and a method for activating it.

## Description

### Field of the Invention

The invention relates to exothermic nonwoven composite material, which may be used as a disposable, replaceable heating pad in devices for heating food, medical compresses, etc.

### Background of the Invention

Exothermic materials are known which have the capability of releasing heat upon contact with air. Exothermic compositions of this type comprise powder of a metal or alloy which is oxidized by air oxygen in the presence of water, and further comprises a neutral salt of a metal, water-retaining agents, catalysts and other additives [US patent 3967049, 1976, IPC² F 24 J 1/00; Japanese patent 58-037075, 1983, IPC³ A 61 F 7/03].

Usually these materials are an internal layer of an article and are enclosed in a bag that is in contact with the object being heated. When such exothermic materials are used, there are problems related to creating an adjustable uniform heating effect over the whole surface of the article to ensuring continuous, unhindered contact with air. There also exists a problem of gas emission when exothermic materials are stored in closed containers without the access of air. Previous designs in this field related to methods of creating articles with uniform distribution of a metal powder, permeable to air and convenient in use.

For example, self-heating cushions for seats were proposed [US patent 4995126, 1991, IPC⁵ A 47 C 21/04, A 61 F 7/08]. A mixture of powders of oxidized metal and water-containing material in air permeable containers is used as a disposable heat pad. In order to create a more or less uniform layer and, correspondingly, uniform heating, special holders are used that fasten the three layers of the article. In order to simplify the unhindered access of air to the exothermic mixture during use of the cushion, a cushioning means is proposed which is made of polyurethane or other polymer foams.

Appliances for heating food, medical compresses, etc., are also known in which an easily corroded powder of a Mg-Fe alloy (Fe content to 5%), activated with an electrolyte solution, is used as the exothermic material. In order to attain uniform distribution in the exothermic layer, the alloy is well mixed with a powder of polyethylene of ultrahigh molecular weight and is baked at 168°C [US patent 4522190, 1985, IPC³ F 24 J 1/00].

However, in that case the surface of a substantial portion of the particles of the exothermic powder is covered with a melt (cake) of the polymer, causing inactivation of the oxidation (corrosion) process and, accordingly, reducing the emission of heat, wherein the inactivation takes place nonuniformly along a cut of the layer.

The authors of US patent 5117809, 1992, IPC⁵ F 24 J 1/00, attempted to avoid these disadvantages and ensure uniform distribution of the layer of the mixture of the Mg-Fe alloy powder, electrolyte and other additives, by placing the exothermic mixture between two layer of polymer materials that are permeable to air.

Problems also arise when powdered iron-containing mixtures and tablets pressed from powders are used in heating devices intended for medical purposes. In particular these problems may be: the rising of dust of finely-dispersed reagents during the mixing and, in view of this, the necessity of adding water, which may cause spontaneous heating of the mixture, the possibility of local overheating because of the high heat-generating capability of the iron-containing and especially magnesium-containing mixtures and the non-uniform distribution of the active component over the plane of the heating device, the necessity for special measures to prevent overheating.

In order to resolve some of the problems indicated above, it is proposed, for example, that modification of a heating device for medical purposes be carried out so that only one of the surfaces of a container containing powder of an exothermic mixture is permeable to air. The surface lying against a person's skin is covered with a layer of a special polymer, impermeable to air, but containing therapeutic medicinal preparations. In this case the temperature in the process of heat emission varies within a narrow range and does not exceed 42°C [British patent 2301433A, 1996, IPC⁶ F 24 J 1/00, A 61 F 7/03].

Thus, the object of the instant invention is to develop such an exothermic material which would make it possible to resolve all of the aforesaid problems.

### Disclosure of the Invention

The present invention relates to a new flexible exothermic unwoven material, in the fibers of the polymer matrix of which there is a filler of iron powder or a mixture of iron powder (as the oxidizable metal) or a mixture of iron powder and water-retaining agents.

Activated carbon of different brands, zeolites, silica gels, polymer foams, etc., which are known to specialists in this field, may be used as the water-retaining agents.

The content of the filler with respect to the polymer may vary within a wide range. Preferably, the ratio of polymer to the filler (by weight) is 1:3 - 1:4.

More concretely, the filler is finely dispersed powder-like metal iron or a powdered mixture of metal iron and activated carbon (DARCO KBB, SKT, AG-3), or said mixture with the addition of vermiculite. The size of the filler particles does not exceed 20 microns.

In addition to the components indicated above, the mixture may additionally comprise water.

Activated carbon and vermiculite, as is known, are sorbents which are capable of "dosing" water for the iron oxidation reaction. Thus, use of these reagents in the composition of the filler makes it possible to increase the duration of heat emission and to maintain the temperature within a sufficiently narrow predetermined interval. Furthermore, the presence of activated carbon and vermiculite in the filler improves the physicomechanical properties of the proposed exothermic nonwoven material (strength, permeability to air, and others).

An example of a filler comprising all of the indicated components is the mixture having the following composition, g:

| | |
|---|---|
| powdered metal iron | 0.717, |
| activated carbon | 0.151, |
| vermiculite | 0.026, |
| water | 0.106. |

The polymer fiber―the base of the nonwoven material―may be, for example, a copolymer of acrylonitrile and methyl acrylate, taken in a ratio of, % by weight:

| | |
|---|---|
| Acrylonitrile | 95, |
| methyl acrylate | 5. |

The present invention also relates to a method of producing the exothermic nonwoven material disclosed above, the method comprising applying the aforesaid filler during the step of forming the fiber and the nonwoven fabric, in particular: the filler is introduced into a cooled polymer solution in a solvent, for example polyacrylonitrile in dimethylformamide, and then the produced suspension is pressed through fillers to form the fiber, during the hardening and "gluing" of which the unwoven material is formed at the receiving device.

When this method is carried out, a uniform distribution of the iron throughout the whole volume of the material is achieved. The necessary contact between the particles of the filler and the working medium (air, water, electrolyte) is ensured in the produced nonwoven material as a result of the high porosity of the filled fibers. At the same time these particles are reliably fixed in the polymer matrix.

Furthermore, the invention relates to a method of activating the exothermic nonwoven composite material described above, which comprises moistening the material by means of a sprayer with an aqueous solution of sodium chloride.

The concentration of the sodium chloride solution may be 0.5-14.5%, preferably 2.5-10.5%, the weight ratio--solution:dry sample--is usually 0.6:1 - 1:1 (here and below % by weight).

After moistening the material with a solution of sodium chloride, heat emission begins 1-2 minutes after contact with air, the duration of the heat emission may be up to 20 hours. The temperature of the material may increase by 5-40°C depending on the content of the filler in the nonwoven material, on the composition of the filler, on the brand of carbon, on the concentration of the chloride solution, on the conditions under which heat emission takes place (in air, in a thermally insulated cell, etc.).

Adjustment of the temperature for samples with one and the same composition of the filler may be carried out by changing the access of air, the concentration of the sodium chloride solution, the number of layers of the nonwoven material.

One of the advantages of the proposed material is that there is no need for special precautionary measures (airtight packaging, etc.) during storage of dry exothermic nonwoven material.

Thus, the proposed material may serve as a disposable replaceable heating element in autonomous heating devices, for example, for heating medicinal preparations, different portions of the body, food and for other domestic and medical purposes. When the proposed exothermic nonwoven composite material is used, the problems related to uniform distribution of heat, safe storage are resolved, and the possibility of adjusting the temperature in the necessary range is provided for.

The examples provided below illustrate the proposed invention, but do not in any way limit it.

### Example 1.

### Sample 10a.

Composition of the filler: finely dispersed powdered ATW-230 iron having particle size less than 20 microns. The content of the filler in the nonwoven material was 60% (ratio polymer: filler was 1:1.5). The surface density (weight per 1 m²) was 500 g/m². Conditions of drying the material after washing off the solvent (DMFA) with water: in a chamber blown through with nitrogen, at a temperature of 80°C for 110 min.

A sample weighing 4 g was moistened with a solution of sodium chloride having a concentration of 14.5% by means of a sprayer; the weight of the solution was 3.5 g. The moist sample was rolled into a roll and placed in a measuring cylindrical cell with thermal insulation and dimensions: diameter 30 mm, height 70 mm. An upper opening of the cell was closed with a film permeable to air. The temperature in the cell was measured after 1 min after the start with an accuracy of 0.1-0.2°C.

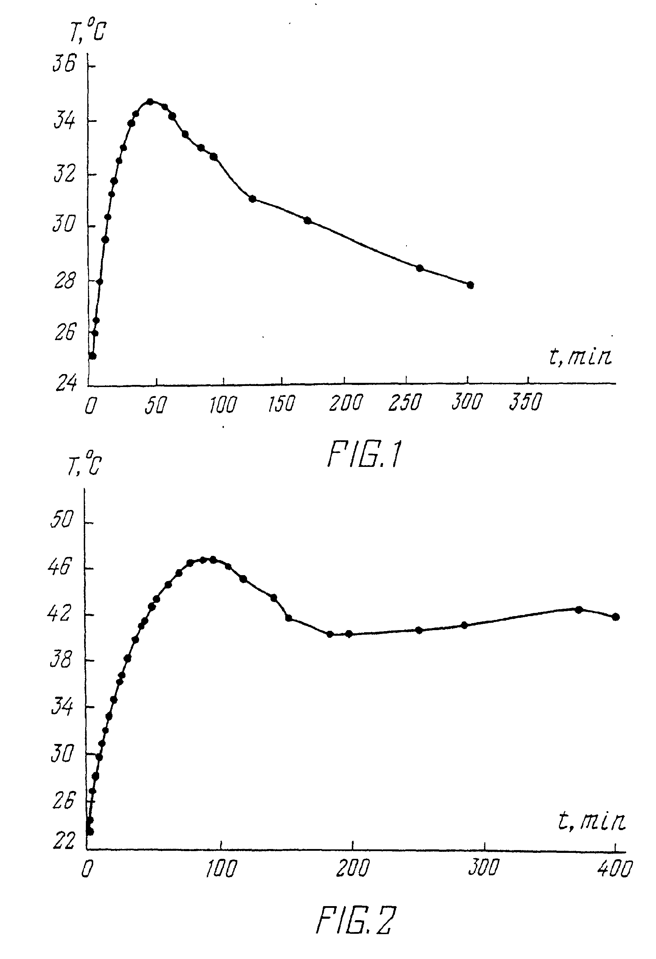

The results of the measurements are presented in the form of a graph showing the relationship between temperature and time (the temperature curve in Fig. 1). The maximum temperature rise Δt max = 10°C, the time at which the maximum temperature (t max) was reached - 45 min, the time during which the temperature remained below the maximum by 4°C ~ 2 hours.

### Example 2.

### Sample 13.

Composition of the filler: powdered iron (as in example 1), activated SKT carbon with particle size not more than 20 microns. The iron: carbon ratio (% by weight) is 82.6:17.4.

The content of the filler was 80% (polymer:filler ratio = 1:4). Surface density - 530 g/m². Dried in air with intensive blowing from two sides at 40°C for 105 min. Weight of sample 6 g, solution of NaCl - 5 g. Activation of the solution of sodium chloride and measurement of the temperature curve carried out according to the method of example 1. The maximum increase of temperature (t max) - 24°C, the time at which t max was reached - 75 min, the time during which the temperature remained below the maximum by 4°C - more than 6 hours (Fig. 2).

### Example 3.

### Sample 6.

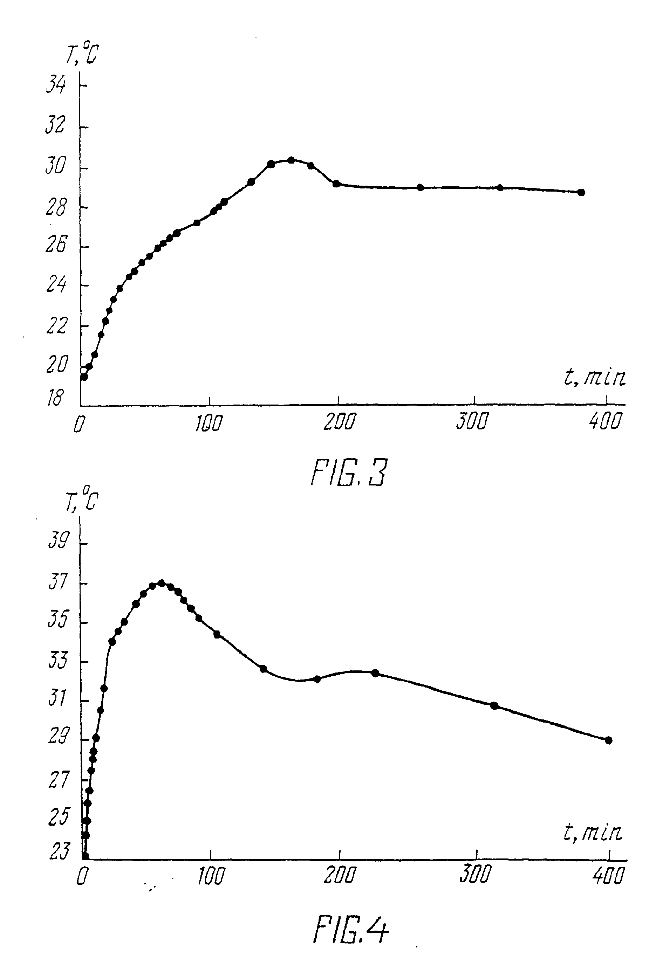

Composition of the filler: powdered iron (as in example 1), activated DARCO KBB carbon and vermiculite having particle size not more than 20 microns. The iron:carbon:vermiculite ratio (% by weight) = 80.2:16.89:2.9. Content of the filler in the sample - 70% (polymer:filler ratio 1:2.33). Surface density - 450 g/m². Sample dried in air. Weight of the sample 6 g, solution of NaCl - 4.5 g. Activation and measurement of temperature curve in accordance with the method of example 1. The maximum increase of temperature (t max) was 11°C, the time at which t max was reached - 160 min The time during which the temperature remained below the maximum by 1°C - more than 6 hours (Fig. 3).

### Example 4.

### Sample 29

Composition of the filler: iron, SKT carbon and vermiculite in the ratio as in example 3. Content of the filler - 80%. Surface density - 570 g/m. Dried in air at a temperature of 35°C for 80 min. Weight of sample - 6.5 g, of solution of NaCl - 5 g. Activation and measurement of temperature curve - in accordance with method of example 1. The maximum increase of temperature (t max) was 14°C, the time at which t max, was reached - 50 min, the time during which the temperature remained below the maximum by 4°C - more than 4 hours (Fig. 4).

### Example 5.

### Sample 8

Composition of the filler: iron, DARCO KBB carbon (as in example 3) and water in a ratio (% by weight) of Fe:C:H₂0 - 73.6:15.5:10.9. Content of filler - 70%. Surface density - 260 g/m². Dried in air at 30°C for 90 min. Weight of sample - 5 g, of solution of NaCI -3.5 g.

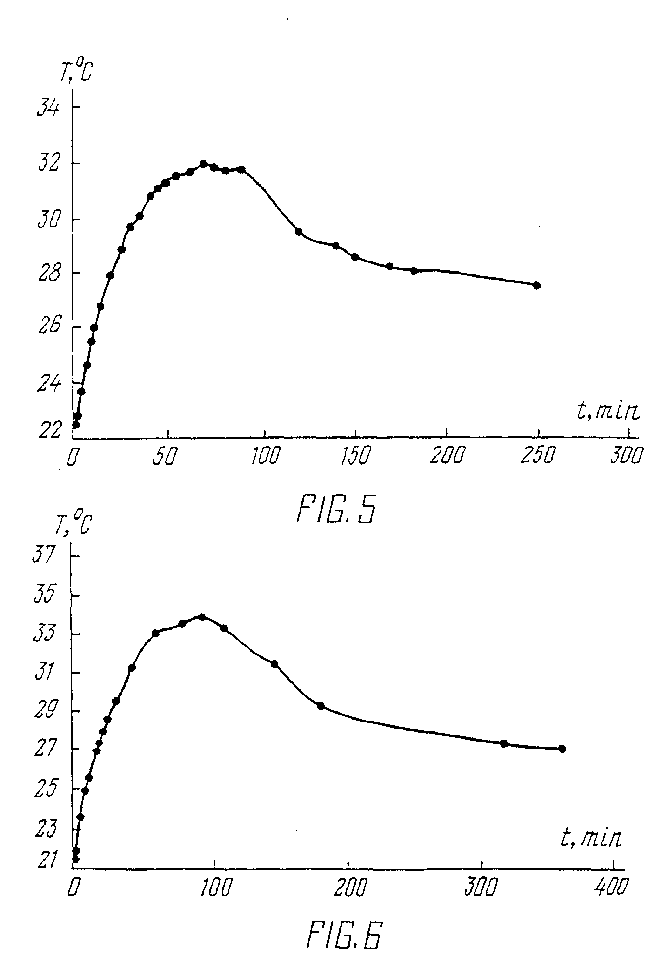

Activation and measurement of temperature curve - in accordance with method of example 1. The maximum increase of temperature (t max) was 10°C, the time at which t max was reached - 60 min, the time during which the temperature remained below the maximum by 4°C - more than 3 hours (Fig. 5).

### Example 6.

### Sample 14

Composition of the filler: iron, DARCO KBB carbon, vermiculite, water. Ratio of components (% by weight), respectively 71.7:15.1:2.6:10.6. Content of filler - 80%. Surface density - 300 g/m². Dried in air at 30°C for 60 min. Weight of sample - 6 g, of solution of NaCl - 5 g. Activation and measurement of temperature curve - in accordance with method of example 1. The maximum increase of temperature (t max) was 12°C, the time at which t max was reached - 90 min, the time during which the temperature remained below the maximum by 4°C - about 3 hours, above the initial temperature by 6°C - more than 6 hours (Fig. 6).

### Example 7. Production of samples of nonwoven composite materials on a base of acrylonitrile polymer fiber with an exothermic filler.

Powder of an acrylonitrile (95%) and methyl acrylate (5%) copolymer - 20 g - is dissolved in dimethylformamide - 180 g - in a laboratory reactor at 90-95°C. The concentration of the polymer in the solution is 11-12%, the specific viscosity of the polymer 1.6-1.8. A filler, preliminarily ground (if necessary) to a particle size of not more than 20 microns in a jet type pulverizer with forced inertial classification of the ground products, is introduced into the polymer solution cooled to 18-20°C. The amount of the filler introduced into the solution is 20-80 g per 20 g of the polymer (weight ratio polymenfiller is 1:1-1:4). After mixing the filler, deaeration of the polymer solution is carried out. The viscosity and stability of the compositions are determined in order to monitor the development of optimum modes. It is shown that it is preferable, from the position of the rheological properties of the polymer solution, to work with iron-carbon and iron-carbon-vermiculite compositions.

Filled fibers are formed from the obtained compositions using the spinning method and, simultaneously, fabric of a fibrous nonwoven material is formed on the surface of a receiving device. The structure of the produced material, which is characterized by the number and strength of the "joints" at the points of intersection of the hardening fibers determines the air-permeability and strength of the product. The strength is characterized by the breaking load for a 20x5 cm strip of material; its value for the produced samples is within the range of from 10 to 130 N. The air-permeability is characterized by the resistance to an air stream; this value varied within the range of from 1 to 20 mm of water. After washing out the solvent with water (~5 1 per sample 26x30 cm in size), the samples are either dried in air with air intensively blown from two sides, or in an inert atmosphere (for example, in a chamber through which nitrogen is blown). These methods of drying make it possible to prevent or substantially reduce the oxidation of iron in the process of producing samples. Monitoring the state of the iron in the fiber of the polymer matrix was carried out by means of gamma-resonance spectra.

### Example 8.

In examples 1-6 presented above, the samples were activated with a solution of sodium chloride at a concentration of 14.5%, since heating at the initial stage is sufficiently rapid at that concentration. The dependence of the main heat emission parameters - Δt max and the time at which t max is reached - on the concentration of the solution of sodium chloride over a wide range of concentrations was measured on samples 39 and 40.

Sample 39. Composition of the filler: iron and SKT carbon (as in example 2), content of the filler - 80%, surface density - 620 g/m². Dried in air with intensive blowing from two sides at 35°C for 60 min. Weight of sample - 6 g, of solution - 5 g.

Sample 40. Composition of the filler and content as for sample 39, surface density - 670 g/m². Dried in air at 40°C for 80 min. Weight of sample - 6.5 g, of solution - 5.2 g.

Activation of samples and measurement of temperature curves were carried out according to the method of example 1. The concentration of the solution varied from 0.1 to 14.5% NaCl.

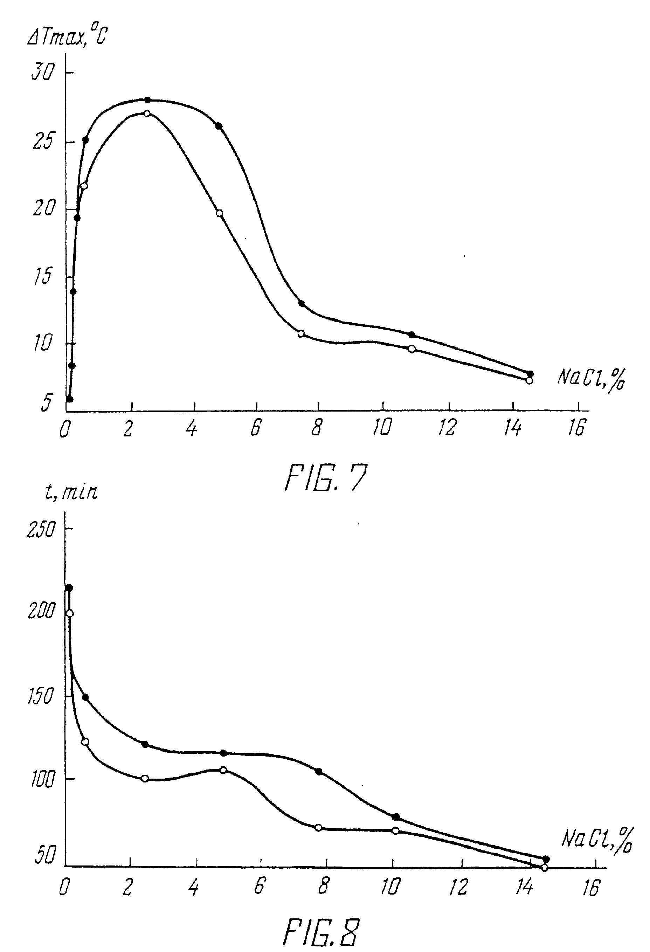

Graphs showing the relationship between the parameters - Δt max and the time at which t max was reached - and the concentration of NaCl are shown in Fig. 7. It follows from Fig. 7 that substantial heat emission occurs when samples are activated with a solution having a concentration > 0.5%. The maximum value of t max is observed in the range of 0.5-5% NaCl. The time at which t max is reached decreases as the concentration of NaCl increases. Obviously, the heat emission can be adjusted by using different concentrations of the solution-activator.

## Claims

1. An exothermic nonwoven composite material on the base of a polymer fiber with a filler capable of releasing heat upon contact with air.

2. The material according to claim 1, **characterized in that** the polymer: filler (weight) ratio is 1:1 - 1:4, more preferably 1:3 - 1:4.

3. The material according to claim 1, **characterized in that** the filler is a finely dispersed powdered metal iron having particle size not more than 20 microns.

4. The material according to claim 3, **characterized in that** the filler additionally comprises activated carbon.

5. The material according to claim 4, **characterized in that** the activated carbon is SKT, AG-3 or DARCO KBB carbon.

6. The material according to claim 4, **characterized in that** the mixture additionally comprises vermiculite.

7. The material according to claims 4-6, **characterized in that** the filler additionally comprises water.

8. The material according to claim 7, **characterized in that** the filler has the following composition, g:
powdered metal iron 0.717,
activated carbon 0.151,
vermiculite 0.026,
water 0.106.

9. The material according to claim 1, **characterized in that** the polymer fiber is a copolymer of acrylonitrile and methyl acrylate, taken in the ratio, % by weight:
acrylonitrile 95,
methyl acrylate 5.

10. A method of preparing the exothermic nonwoven composite material **characterized in** claim 1, comprising introducing a filler into a cooled solution of a polymer in a solvent, for example a solution of polyacrylonitrile in dimethylformamide, and then forming fibers by means of the spinning method, which fibers when glued form the nonwoven material.

11. A method of activating the exothermic nonwoven composite material **characterized in** claim 1, comprising moistening the material with an aqueous solution of a metal chloride, for example sodium chloride.

12. The method according to claim 10, **characterized in that** the aqueous solution of sodium chloride has a concentration of 0.5-14.5%, preferably 2.5-10.5%.
